(19) Europäisches Patentamt
European Patent Office
Office européen des brevets

(11) Veröffentlichungsnummer : **0 409 077 B1**

(12) # EUROPÄISCHE PATENTSCHRIFT

(45) Veröffentlichungstag der Patentschrift : **20.10.93 Patentblatt 93/42**

(21) Anmeldenummer : **90113327.2**

(22) Anmeldetag : **12.07.90**

(51) Int. Cl.5 : **C07C 251/32,** C07C 251/40, C07C 251/52, C07C 251/66, C07C 251/68, C07C 251/80, C07C 281/10, C07D 213/53, C07C 47/20, A01N 35/10

(54) **1-Aza-butadiene und diese Verbindungen enthaltende Fungizide.**

(30) Priorität : **19.07.89 DE 3923896**

(43) Veröffentlichungstag der Anmeldung : **23.01.91 Patentblatt 91/04**

(45) Bekanntmachung des Hinweises auf die Patenterteilung : **20.10.93 Patentblatt 93/42**

(84) Benannte Vertragsstaaten : **AT BE CH DE DK ES FR GB GR IT LI NL SE**

(56) Entgegenhaltungen :
**EP-A- 0 040 583**
**EP-A- 0 113 106**
**DE-A- 2 507 685**
**DE-A- 3 118 656**

(73) Patentinhaber : **BASF Aktiengesellschaft**
**Carl-Bosch-Strasse 38**
**D-67063 Ludwigshafen (DE)**

(72) Erfinder : **Lauer, Manfred, Dr.**
**Im Zinkig 114**
**D-6700 Ludwigshafen (DE)**
Erfinder : **Zipperer, Bernhard, Dr.**
**Am Herrgottsacker 6**
**D-6716 Dirmstein (DE)**
Erfinder : **Goetz, Norbert, Dr.**
**Schoefferstrasse 25**
**D-6520 Worms 1 (DE)**
Erfinder : **Lorenz, Gisela, Dr.**
**Erlenweg 13**
**D-6730 Neustadt (DE)**
Erfinder : **Ammermann, Eberhard, Dr.**
**Sachsenstrasse 3**
**D-6700 Ludwigshafen (DE)**

**EP 0 409 077 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft 1-Aza-butadiene und diese enthaltende Fungizide.

Es wurden neue 1-Aza-butadiene der Formel I gefunden

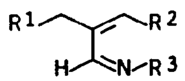

I

in der

$R^1$ und $R^2$ gleich oder verschieden sind und Phenyl, Biphenyl, Naphthyl oder Pyridyl bedeuten, wobei diese Reste einfach bis dreifach durch Halogen, Nitro, Phenoxy, Amino, Haloalkyl, Alkyl oder Alkoxy mit jeweils 1 bis 4 C-Atomen substituiert sein können, oder einen linearen oder verzweigten $C_1$-$C_8$-Alkylrest bedeuten, der durch einen gegebenenfalls substituierten Phenylrest substituiert ist,

$R^3$ einen gegebenenfalls substituierten Phenylrest oder den Rest $OR^4$ bedeutet,

$R^4$ Wasserstoff, einen gegebenenfalls verzweigten $C_1$-$C_6$-Alkylrest oder eine aromatische oder aliphatische Acylgruppe bedeutet oder $R^3$ den Rest $NHR^5$ bedeutet,

$R^5$ Wasserstoff oder einen gegebenenfalls substituierten Phenylrest oder die Carbamid-Gruppe $CONH_2$ bedeutet und zusätzlich die Verbindungen, in denen $R^1$ tertiär-Butyl, $R^2$ Cyclohexyl und $R^3$ OH, OMe, OEt, O-CO-Me, O-CO-Phenyl, NH-Phenyl oder 4-Br-Phenyl bedeuten, die eine sehr gute fungizide Wirkung haben.

$R^1$ und $R^2$ in der Formel I bedeuten beispielsweise unabhängig voneinander 1-Naphthyl, 2-Naphthyl, 2-Pyridyl, 3-Pyridyl, 4-Pyridyl, p-Biphenyl, Phenyl, Halogenphenyl, 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, Halo-$C_1$-$C_4$-alkylphenyl, 2-Trifluormethylphenyl, 3-Trifluormethylphenyl, 4-Trifluormethylphenyl, $C_1$-$C_4$-Alkylphenyl, 2-Methylphenyl, $C_1$-$C_4$-Alkoxyphenyl, 3-Methylphenyl, 4-Methylphenyl, 2-Methoxyphenyl, 3-Methoxyphenyl, 4-Methoxyphenyl, 2,3-Dichlorphenyl, 2,4-Dichlorphenyl, 2,5-Dichlorphenyl, 2,6-Dichlorphenyl, 2-Chlor-6-fluorphenyl, 2-Chlor-4-fluorphenyl, 2-Fluor-4-chlorphenyl, 2,4-Dimethylphenyl, 2,6-Dimethylphenyl oder 2,4,6-Trimethylphenyl oder $C_1$-$C_8$-Alkyl, insbesondere $C_1$-$C_4$-Alkyl, Methyl, Ethyl, Propyl, i-Propyl, Butyl, s-Butyl, t-Butyl, Pentyl, 2,2-Dimethylpropyl, Benzyl, Halogenbenzyl, 4-Chlorbenzyl, 4-Fluorbenzyl, $C_1$-$C_4$-Alkylbenzyl, 4-Methylbenzyl, 2-Chlorbenzyl, 2-Fluorbenzyl, 2-Methylbenzyl, 2-Phenylethyl, Halogenphenylethyl, 4-Chlor-phenylethyl, 4-Fluor-phenylethyl oder 3-Phenylpropyl.

Besonders bevorzugt sind Verbindungen, in denen $R^1$ für einen gegebenenfalls substituierten Phenylring steht, beispielsweise Phenyl, Halogenphenyl, 4-Chlorphenyl, 4-Flurophenyl, 2-Chlorphenyl, 2-Fluorphenyl, und $R^2$ für 2,2-Dimethylpropyl oder 3,3-Dimethylbutyl oder für einen gegebenenfalls substituierten Phenylrest steht wie beispielsweise Phenyl, Halogenphenyl, 4-Chlorphenyl, 4-Fluorphenyl, 2-Chlorphenyl oder 2-Fluorphenyl.

$R^3$ bedeutet beispielsweise die Hydroxylgruppe oder die Gruppe $OR^4$, wobei $R^4$ für einen aliphatischen oder aromatischen Acylrest wie beispielsweise $C_1$-$C_4$-Alkyl-CO, Acetyl, Propionyl, Butyryl, Benzoyl oder Halogenbenzoyl, 4-Chlorbenzoyl steht oder eine gegebenenfalls substituierte $C_1$-$C_6$-Alkylgruppe, insbesondere $C_1$-$C_4$-Alkylgruppe bedeutet wie Methyl, Ethyl, Propyl, Butyl, Benzyl oder Halogenbenzyl oder

$R^3$ bedeutet den Rest $NHR^5$, in dem $R^5$ Wasserstoff, die Carbamidgruppe $CONH_2$ oder ggf. substituiertes Phenyl bedeutet, beispielsweise Phenyl, Nitrophenyl, 4-Nitrophenyl oder 2,4-Dinitrophenyl.

Schließlich steht $R^3$ auch für gegebenenfalls substituiertes Phenyl, beispielsweise Phenyl, Halogenphenyl, 4-Chlorphenyl, 4-Bromphenyl oder $C_1$-$C_4$-Alkylphenyl, 4-Methylphenyl.

Besonders bevorzugt für $R^3$ sind die Hydroxylgruppe und davon abgeleitete Derivate, die durch Alkylierung oder Acylierung erhalten werden können.

1-Aza-Butadiene der Formel I können erhalten werden z.B. durch Umsetzung der Aldehyde II

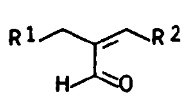

II

mit Verbindungen der Formel $H_2N$-$R^3$ gegebenenfalls in Gegenwart eines Katalysators und eines geeigneten Lösungsmittels. Als Katalysatoren können wasserentziehende Mittel wie Molekularsiebe, Magnesiumsulfat, Natriumsulfat oder Calciumchlorid verwendet werden, aber auch Säuren wie Schwefelsäure, Salzsäure oder

Essigsäure oder aber basische Verbindungen wie Natriumhydroxid, Kaliumhydroxid, Kaliumcarbonat oder Kaliumacetat. Als Lösungsmittel dienen beispielsweise niedere Alkohole wie Methylalkohol, Ethylalkohol und ihre Mischungen mit Wasser wie auch Propylalkohol, Isopropylalkohol, Butanol, s-Butanol, t-Butanol, Ketone wie Aceton, Ether wie Diethylether, Tetrahydrofuran oder Methyl-tert.-butylether sowie Methylenchlorid, aromatische Kohlenwasserstoffe wie Toluol oder Xylol sowie Dimethylformamid oder Dimethylsulfoxid.

Vorschrift 1

5,5-Dimethyl-2-formyl-1-(p-chlorphenyl)-hexen-(2) (Verbindung 136a)

Zu einer Lösung von 10,5 g 50 %iger (Gew.-%) wäßriger NaOH in 500 ml Methanol werden 79 g (0,79 M) 3,3-Dimethylbutyraldehyd zugegeben und anschließend bei 34 °C 123 g (0,73 M) p-Chlor-dihydrozimtaldehyd zugetropft. Nach 1stündigem Nachrühren wird mit verdünnter Schwefelsäure neutralisiert und nach Einengen der Lösung fraktionierend destilliert.

Kp 119-122°C/0,01 mbar, Ausbeute 115,9 g (59 % d. Th.)

Vorschrift 2

1-(o-Chlorphenyl-3(p-Fluorphenyl)-2-formyl-propen(1)

Zu einer Lösung von 4 g 50 %iger Natronlauge in 300 ml Methanol werden 71,7 g (0,51 M) 2-Chlorbenzaldehyd unter Kühlung so zugegeben, daß die Temperatur nicht über 20 °C ansteigt. Anschließend werden bei 25-30°C 70,0 g (0,46 M) p-Fluor-dihydrozimtaldehyd innerhalb von 4 Stunden zugetropft. Man rührt eine Stunde bei 30°C nach, neutralisiert mit verdünnter Schwefelsäure, engt ein und destilliert.

Kp 164-184°C/0,3 mbar; Ausbeute 97,8 g (77 % d. Th.)

Vorschrift 3

4,4-Dimethyl-2-formyl-1-(p-Chlorphenyl)-penten(1) (Verbindung 63a)

Zu einer Lösung von 15,8 g 50 %iger Natronlauge in 500 ml Methanol werden 134 g (0,95 M) 4-Chlorbenzaldehyd zugegeben und anschließend 90 g (0,79 M) 4,4-Dimethylpentanal bei 35 °C im Verlauf von 3 Stunden zugetropft. Nach dreistündigem Nachrühren wird mit verdünnter Schwefelsäure neutralisiert, eingeengt und destilliert.

Kp 117-120°C/0,4 mbar; Ausbeute 83,5 g (39 % d. Th.)

Herstellungsbeispiel 1 (Verbindung 136)

Zu einer Lösung von 7,5 g (0,03 M) des Aldehyds aus Vorschrift 1 in 100 ml Methanol wurden 4,2 g (0,06 M) Hydroxylammoniumhydrochlorid gelöst in einer Methanol/Wasser-Mischung und 4,1 g (0,03 M) Kalimcarbonat zugegeben. Nach 1stündiger Reaktionszeit bei 25°C wurde eingeengt und das Produkt mit Methylenchlorid extrahiert.

Ausbeute 6,8 g (85 % d. Th.), Schmp. 78°C

Herstellungsbeispiel 2 (Verbindung 139)

5,0 g (0,019 M) des Oxims aus Beispiel 1 wurden in 50 ml Tetrahydrofuran gelöst und nach Zugabe von 3,0 g Pyridin und 0,1 g 4-(N,N-Dimethylamino)pyridin wurden bei 50°C 2,2 g (0,03 M) Acetylchlorid gelöst in 20 ml Tetrahydrofuran zugetropft. Nach 72stündiger Reaktionszeit bei 25°C wurde eingeengt, mit Methyl-tert.-butylether aufgenommen, mit verdünnter $NaHCO_3$-Lösung gewaschen, die organische Phase getrocknet und eingeengt.

Ausbeute 4,3 g (78 % d. Th.) IR-Banden, 2958, 1769, 1492, 1366, 1204 $cm^{-3}$

In entsprechender Weise können die folgenden Verbindungen hergestellt werden.

Tabelle 1

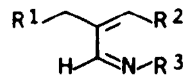

| Nr. | $R^1$ | $R^2$ | $R^3$ | Schmp (°C)/IR ($cm^{-1}$)/ (NMR) |
|---|---|---|---|---|
| 1 | $n-C_3H_7$ | Phenyl | OH | |
| 2 | $n-C_3H_7$ | 4-Cl-Ph | OH | |
| 3 | $n-C_3H_7$ | 4-F-Ph | OH | 91- 93 |
| 4 | $n-C_3H_7$ | 4-F-Ph | NH-CONH2 | 152 |
| 5 | $n-C_3H_7$ | 4-Me-Ph | OH | |
| 6 | $n-C_3H_7$ | 2-Cl-Ph | OH | |
| 7 | $n-C_3H_7$ | 2-F-Ph | OH | |
| 8 | $n-C_3H_7$ | $2,4-Cl_2Ph$ | OH | |
| 9 | $n-C_3H_7$ | 3-Pyridin | OH | 118-121 |
| 10 | $n-C_3H_7$ | 3-Pyridin | OMe | |
| 11 | $n-C_3H_7$ | 3-Pyridin | OEt | |
| 12 | $n-C_3H_7$ | 3-Pyridin | O-CO-Me | 0,9-2,6 (9, m); 2,2 (3,s); 6,8-8,7 (6, m) |
| 13 | $n-C_3H_7$ | 3-Pyridin | O-CO-Ph | 0,9-2,7 (9, m); 6,8-8,7 (11, m) |
| 14 | $n-C_3H_7$ | 4-Pyridin | OH | |
| 15 | $n-C_3H_7$ | 2-Pyridin | OH | |
| 16 | $n-C_3H_7$ | 1-Naphthyl | OH | |
| 17 | $n-C_3H_7$ | 2-Naphthyl | OH | |
| 18 | $n-C_3H_7$ | 4-Biphenyl | OH | |
| 19 | n-Bu | Phenyl | OH | |
| 20 | n-Bu | 4-Cl-Ph | OH | |
| 21 | n-Bu | 4-F-Ph | OH | |
| 22 | n-Bu | 4-Me-Ph | OH | |
| 23 | n-Bu | 2-Cl-Ph | OH | |
| 24 | n-Bu | 2-F-Ph | OH | |
| 25 | n-Bu | $2,4-Cl_2-Ph$ | OH | |
| 26 | n-Bu | 3-Pyridin | OH | |
| 27 | t-Bu | Ph | OH | |
| 28 | t-Bu | 4-Cl-Ph | OH | 108-110 |
| 29 | t-Bu | 4-Cl-Ph | OMe | |
| 30 | t-Bu | 4-Cl-Ph | OEt | 2959, 1490, 1092, 1055 |
| 31 | t-Bu | 4-Cl-Ph | O-CO-Me | 2961, 1769, 1490, 1203 |
| 32 | t-Bu | 4-Cl-Ph | O-CO-Ph | |
| 33 | t-Bu | 4-Cl-Ph | NH-Ph | |

Tabelle 1 (Fortsetzung)

| Nr. | $R^1$ | $R^2$ | $R^3$ | Schmp (°C)/IR ($cm^{-1}$)/ (NMR) |
|---|---|---|---|---|
| 34 | t-Bu | 4-F-Ph | OH | |
| 35 | t-Bu | 4-F-Ph | O-CO-Me | |
| 36 | t-Bu | 4-Me-Ph | OH | |
| 37 | t-Bu | 2-Cl-Ph | OH | |
| 38 | t-Bu | 2-F-Ph | OH | |
| 39 | t-Bu | 2,4-$Cl_2$-Ph | OH | |
| 40 | t-Bu | 3-Pyridin | OH | 178 |
| 41 | t-Bu | 3-Pyridin | O-CO-Me | 0,8-2,7 (14,m); 6,9-8,7 (6,m) |
| 42 | t-Bu | Cyclohexyl | OH | 88 |
| 43 | t-Bu | Cyclohexyl | OMe | 2930, 2852, 1448, 1059 |
| 44 | t-Bu | Cyclohexyl | OEt | 2929, 2852, 1448, 1056 |
| 45 | t-Bu | Cyclohexyl | O-CO-Me | 2930, 2853, 1773, 1366, 1202 |
| 46 | t-Bu | Cyclohexyl | O-CO-Ph | < 50 |
| 47 | t-Bu | Cyclohexyl | NH-Ph | 123 |
| 48 | t-Bu | Cyclohexyl | 4-Br-Ph | 2928, 2852, 1612, 1481, 825 |
| 49 | n-Pentyl | Ph | OH | |
| 50 | n-Pentyl | 4-Cl-Ph | OH | |
| 51 | n-Pentyl | 4-F-Ph | OH | |
| 52 | n-Pentyl | 4-Me-Ph | OH | |
| 53 | n-Pentyl | 2-Cl-Ph | OH | |
| 54 | n-Pentyl | 2-F-Ph | OH | |
| 55 | n-Pentyl | 2,4-$Cl_2$-PH | OH | |
| 56 | n-Pentyl | 3-Pyridin | OH | 120 |
| 57 | n-Pentyl | 3-Pyridin | OMe | 0,9-3,9 (16,m); 6,5-8,6 (6,m) |
| 58 | n-Pentyl | 3-Pyridin | OEt | 0,9-4,2 (18,m); 6,5-8,6 (6,m) |
| 59 | n-Pentyl | 3-Pyridin | O-CO-Me | 0,9-2,6 (16,m); 6,4-8,7 (6,m) |
| 60 | n-Pentyl | 3-Pyridin | O-CO-Ph | 0,9-2,8 (13,m); 6,7-8,7 (11,m) |
| 61 | n-Pentyl | 3-Pyridin | NH-Ph | 0,9-2,7 (13,m); 6,4-8,6 (11,m) |
| 62 | $CH_2$+ | Ph | OH | |
| 63 | $CH_2$+ | 4-Cl-Ph | OH | |
| 64 | $CH_2$+ | 4-F-Ph | OH | |
| 65 | $CH_2$+ | 4-Me-Ph | OH | |
| 66 | $CH_2$+ | 2-Cl-Ph | OH | |
| 67 | $CH_2$+ | 2-F-Ph | OH | |
| 68 | $CH_2$+ | 2,4-$Cl_2$-Ph | OH | |
| 69 | $CH_2$+ | 3-Pyridin | OH | |
| 70 | n-Hexyl | Ph | OH | |
| 71 | n-Hexyl | 4-Cl-Ph | OH | |
| 72 | n-Hexyl | 4-F-Ph | OH | |

Tabelle 1 (Fortsetzung)

| Nr. | $R^1$ | $R^2$ | $R^3$ | Schmp (°C)/IR ($cm^{-1}$)/ (NMR) |
|---|---|---|---|---|
| 73 | n-Hexyl | 4-Me-Ph | OH | |
| 74 | n-Hexyl | 2-Cl-Ph | OH | |
| 75 | n-Hexyl | 2-F-Ph | OH | |
| 76 | n-Hexyl | 2,4-$Cl_2$-Ph | OH | |
| 77 | n-Hexyl | 3-Pyridin | OH | |
| 78 | $CH_2$-$CH_2$+ | Ph | OH | |
| 79 | $CH_2$-$CH_2$+ | 4-Cl-Ph | OH | |
| 80 | $CH_2$-$CH_2$+ | 4-F-Ph | OH | |
| 81 | $CH_2$-$CH_2$+ | 4-Me-Ph | OH | |
| 82 | $CH_2$-$CH_2$+ | 2-Cl-Ph | OH | |
| 83 | $CH_2$-$CH_2$+ | 2-F-Ph | OH | |
| 84 | $CH_2$-$CH_2$+ | 2,4-$Cl_2$-Ph | OH | |
| 85 | $CH_2$-$CH_2$+ | 3-Pyridin | OH | |
| 86 | Ph | Ph | OH | |
| 87 | Ph | 4-Cl-Ph | OH | |
| 88 | Ph | 4-F-Ph | OH | |
| 89 | Ph | 4-Me-Ph | OH | |
| 90 | Ph | 2-Cl-Ph | OH | |
| 91 | Ph | 2-F-Ph | OH | |
| 92 | Ph | 2,4-$Cl_2$-Ph | OH | |
| 93 | Ph | 3-Pyridin | OH | |
| 94 | Ph | n-Bu | OH | |
| 95 | Ph | t-Bu | OH | |
| 96 | Ph | n-Pentyl | OH | |
| 97 | Ph | $CH_2$+ | OH | |
| 98 | Ph | $CH_2$+ | OMe | |
| 99 | Ph | $CH_2$+ | OEt | |
| 100 | Ph | $CH_2$+ | O-CO-Me | |
| 101 | Ph | $CH_2$+ | O-CO-Ph | |
| 102 | Ph | $CH_2$+ | NH-Ph | |
| 103 | Ph | $CH_2$+ | 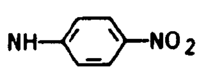 | |
| 104 | 4-Cl-Ph | $CH_2$+ | NH-CO-$NH_2$ | 0,9-4,1 (13,m); 6,0-7,5 (6,m) |
| 105 | Ph | n-Hexyl | OH | |
| 106 | Ph | $CH_2$-$CH_2$+ | OH | |
| 107 | Ph | $CH_2$-$CH_2$+ | OMe | |
| 108 | Ph | $CH_2$-$CH_2$+ | OEt | 2956, 2867, 1052, 698 |

Tabelle 1 (Fortsetzung)

| Nr. | $R^1$ | $R^2$ | $R^3$ | Schmp (°C)/IR ($cm^{-1}$)/ (NMR) |
|---|---|---|---|---|
| 109 | Ph | $CH_2$-$CH_2$+ | O-CO-Me | 2956, 2866, 1770, 1366, 1204 |
| 110 | Ph | $CH_2$-$CH_2$+ | O-CO-Ph | 2956, 2907, 1749, 1257, 1245 |
| 111 | Ph | $CH_2$-$CH_2$+ | NH-Ph | 2955, 2904, 2865, 1600, 1495 |
| 112 | Ph | $CH_2$-$CH_2$ | 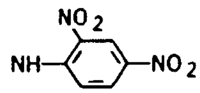 | 145-148 |
| 113 | Ph | $CH_2$-$CH_2$+ | NH-CO-$NH_2$ | 2955, 2907, 1692, 1582, 698 |
| 114 | Ph | $CH_2$-$CH_2$-Ph | OH | 3026, 2925, 1495, 1452, 698 |
| 115 | Ph | $CH_2$-$CH_2$-Ph | OMe | 2935, 1495, 1452, 1047, 698 |
| 116 | Ph | $CH_2$-$CH_2$-Ph | OEt | 2931, 1495, 1453, 1051, 698 |
| 117 | Ph | $CH_2$-$CH_2$-Ph | O-CO-Me | 3026, 1766, 1453, 1204, 700 |
| 118 | Ph | $CH_2$-$CH_2$-Ph | O-CO-Ph | 1746, 1451, 1257, 1246, 700 |
| 119 | Ph | $CH_2$-$CH_2$-Ph | NH-Ph | 3025, 1602, 1494, 1258, 697 |
| 120 | Ph | $CH_2$-$CH_2$-Ph | 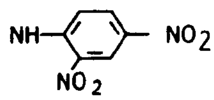 | 144-146 |
| 121 | Ph | $CH_2$-$CH_2$-Ph | NH-CO-$NH_2$ | 3463, 1693, 1582, 696 |
| 122 | 4-Cl-Ph | Ph | OH | |
| 123 | 4-Cl-Ph | 4-Cl-Ph | OH | |
| 124 | 4-Cl-Ph | 4-F-Ph | OH | 152-154 |
| 125 | 4-Cl-Ph | 4-F-Ph | $OCH_3$ | 3,7-3,9 (5,m); 6,8-7,9 (10,m) |
| 126 | 4-Cl-Ph | 4-F-Ph | OEt | 1,1-4,2 (7,m); 6,7-7,9 (10,m) |
| 127 | 4-Cl-Ph | 4-F-Ph | O-CO-Me | 2,2-4,0 (5,m); 7,0-8,2 (10,m) |
| 128 | 4-Cl-Ph | 4-F-Ph | O-CO-Ph | 88 |
| 129 | 4-Cl-Ph | 4-F-Ph | NH-Ph | 142-144 |
| 130 | 4-Cl-Ph | 4-F-Ph | 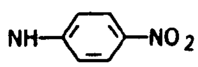 | 195-197 |
| 131 | 4-Cl-Ph | 4-F-Ph | NH-CO-$NH_2$ | 163-164 |
| 132 | 4-Cl-Ph | 4-Me-Ph | OH | |
| 133 | 4-Cl-Ph | 2-Cl-Ph | OH | |
| 134 | 4-Cl-Ph | 2-F-Ph | OH | |
| 135 | 4-Cl-Ph | 2,4-$Cl_2$-Ph | OH | |
| 136 | 4-Cl-Ph | $CH_2$+ | OH | 78 |
| 137 | 4-Cl-Ph | $CH_2$+ | OMe | 2958, 2900, 1492, 1052, 797 |

Tabelle 1 (Fortsetzung)

| Nr. | $R^1$ | $R^2$ | $R^3$ | Schmp (°C)/IR ($cm^{-1}$)/ (NMR) |
|---|---|---|---|---|
| 138 | 4-Cl-Ph | $CH_2$+ | OEt | 0,9-4,1 (16,m); 3,7 (2,s); 5,9-7,8 (6,m) |
| 139 | 4-Cl-Ph | $CH_2$+ | O-CO-Me | 2958, 1769, 1492, 1366, 1204 |
| 140 | 4-Cl-Ph | $CH_2$+ | O-CO-Ph | 2958, 1748, 1491, 1244, 707 |
| 141 | 4-Cl-Ph | $CH_2$+ | NH-Ph | 0,9-3,8 (13,m); 5,8-7,4 (11,m) |
| 142 | 4-Cl-Ph | $CH_2$ | 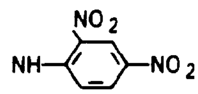 | 154 |
| 143 | 4-Cl-Ph | $CH_2$ | $NH-CO-NH_2$ | 118 |
| 144 | 4-Cl-Ph | $CH_2$+ | O-CO-OEt | |
| 145 | 4-Cl-Ph | $CH_2-CH_2$+ | OH | |
| 146 | 4-Cl-Ph | $CH_2-CH_2$+ | OMe | |
| 147 | 4-Cl-Ph | $CH_2-CH_2$+ | OEt | |
| 148 | 4-Cl-Ph | $CH_2-CH_2$+ | O-CO-Me | |
| 149 | 4-Cl-Ph | $CH_2-CH_2$+ | O-CO-Ph | |
| 150 | 4-Cl-Ph | $CH_2-CH_2$+ | NH-Ph | |
| 151 | 4-Cl-Ph | $CH_2-CH_2$+ | 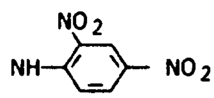 | |
| 152 | 4-Cl-Ph | $CH_2-CH_2$+ | $NH-CO-NH_2$ | |
| 153 | 4-F-Ph | Ph | OH | |
| 154 | 4-F-Ph | 4-Cl-Ph | OH | |
| 155 | 4-F-Ph | 4-F-Ph | OH | |
| 156 | 4-F-Ph | 4-Me-Ph | OH | |
| 157 | 4-F-Ph | 2-Cl-Ph | OH | 3280, 1506, 1432, 1219 |
| 158 | 4-F-Ph | 2-Cl-Ph | $OCH_3$ | 1508, 1218, 977, 761, 532 |
| 159 | 4-F-Ph | 2-Cl-Ph | OEt | |
| 160 | 4-F-Ph | 2-Cl-Ph | O-CO-Me | 2,1-3,9 (5,m); 6,9-8,2 (10,m) |
| 161 | 4-F-Ph | 2-Cl-Ph | O-CO-Ph | 1691, 1510, 1213, 710 |

Tabelle 1 (Fortsetzung)

| Nr. | $R^1$ | $R^2$ | $R^3$ | Schmp (°C)/IR ($cm^{-1}$)/ (NMR) |
|---|---|---|---|---|
| 162 | 4-F-Ph | 2-Cl-Ph | 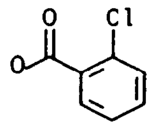 | 1508, 1235, 1036, 749 |
| 163 | 4-F-Ph | 2-Cl-Ph | 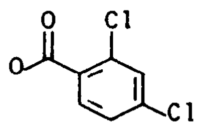 | |
| 164 | 4-F-Ph | 2-Cl-Ph | 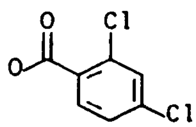 | 1508, 1232, 1037, 758 |
| 165 | 4-F-Ph | 2-Cl-Ph | 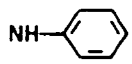 | 1603, 1507, 1258, 751 |
| 166 | 4-F-Ph | 2-Cl-Ph | 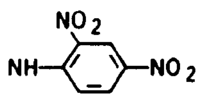 | 188 |
| 167 | 4-F-Ph | 2-Cl-Ph | Phenyl | |
| 168 | 4-F-Ph | 2-Cl-Ph | 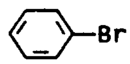 | 78 |
| 169 | 4-F-Ph | 2-Cl-Ph | NH-CO-$NH_2$ | |
| 170 | 4-F-Ph | 2-Cl-Ph | O-$CH_2$-Ph | |
| 171 | 4-F-Ph | 2-Cl-Ph | O-CO-OEt | |
| 172 | 4-F-Ph | 2-F-Ph | OH | |
| 173 | 4-F-Ph | 2-F-Ph | OH | |
| 174 | 4-F-Ph | $CH_2$+ | OH | 0,9-3,7 (13,m); 5,3-7,8 (6,m) |
| 175 | 4-F-Ph | $CH_2$+ | OMe | 2958, 1508, 1224, 1051 |
| 176 | 4-F-Ph | $CH_2$+ | OEt | 2959, 1508, 1223, 1052 |
| 177 | 4-F-Ph | $CH_2$+ | O-CO-Me | 2958, 1769, 1509, 1222, 1204 |
| 178 | 4-F-Ph | $CH_2$+ | O-CO-Ph | 2957, 1748, 1509, 1245, 708 |
| 179 | 4-F-Ph | $CH_2$+ | NH-Ph | 70 |

Tabelle 1 (Fortsetzung)

| Nr. | $R^1$ | $R^2$ | $R^3$ | Schmp (°C)/IR ($cm^{-1}$)/ (NMR) |
|---|---|---|---|---|
| 180 | 4-F-Ph | $CH_2$+ | 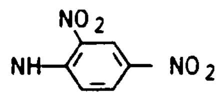 | 161 |
| 181 | 4-F-Ph | $CH_2$+ | $NH-CO-NH_2$ | 53 |
| 182 | 4-F-Ph | $CH_2$+ | Phenyl | |
| 183 | 4-F-Ph | $CH_2$+ | 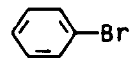 | 2958, 1507, 1481, 1222 |
| 184 | 4-F-Ph | $CH_2-CH_2$+ | OH | |
| 185 | 4-F-Ph | $CH_2-CH_2$+ | OMe | |
| 186 | 4-F-Ph | $CH_2-CH_2$+ | OEt | |
| 187 | 4-F-Ph | $CH_2-CH_2$+ | O-CO-Me | |
| 188 | 4-F-Ph | $CH_2-CH_2$+ | O-CO-Ph | |
| 189 | 4-F-Ph | $CH_2-CH_2$+ | NH-Ph | |
| 190 | 4-F-Ph | $CH_2-CH_2$+ | 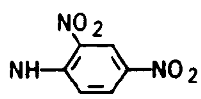 | |
| 191 | 4-F-Ph | $CH_2-CH_2$+ | $NH-CO-NH_2$ | |
| 192 | 2-Cl-Ph | Ph | OH | |
| 193 | 2-Cl-Ph | 4-Cl-Ph | OH | |
| 194 | 2-Cl-Ph | 4-F-Ph | OH | |
| 195 | 2-Cl-Ph | 4-Me-Ph | OH | |
| 196 | 2-Cl-Ph | 2-Cl-Ph | OH | |
| 197 | 2-Cl-Ph | 2-F-Ph | OH | |
| 198 | 2-Cl-Ph | $2,4-Cl_2-Ph$ | OH | |
| 199 | 2-F-Ph | Ph | OH | |
| 200 | 2-F-Ph | 4-Cl-Ph | OH | |
| 201 | 2-F-Ph | 4-F-Ph | OH | |
| 202 | 2-F-Ph | 4-Me-Ph | OH | |
| 203 | 2-F-Ph | 2-Cl-Ph | OH | |
| 204 | 2-F-Ph | 2-F-Ph | OH | |
| 205 | 2-F-Ph | $2,4-Cl_2-Ph$ | OH | |

+ = 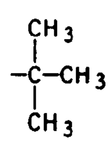

Tabelle 2

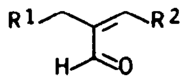

| Nr. | $R^1$ | $R^2$ |
|---|---|---|
| 1a | n-$C_3H_7$ | Phenyl |
| 2a | n-$C_3H_7$ | 4-Cl-Ph |
| 3a | n-$C_3H_7$ | 4-F-Ph |
| 4a | n-$C_3H_7$ | 4-F-Ph |
| 5a | n-$C_3H_7$ | 4-Me-Ph |
| 6a | n-$C_3H_7$ | 2-Cl-Ph |
| 7a | n-$C_3H_7$ | 2-F-Ph |
| 8a | n-$C_3H_7$ | 2,4-$Cl_2$Ph |
| 9a | n-$C_3H_7$ | 3-Pyridin |
| 10a | n-$C_3H_7$ | 3-Pyridin |
| 11a | n-$C_3H_7$ | 3-Pyridin |
| 12a | n-$C_3H_7$ | 3-Pyridin |
| 13a | n-$C_3H_7$ | 3-Pyridin |
| 14a | n-$C_3H_7$ | 4-Pyridin |
| 15a | n-$C_3H_7$ | 2-Pyridin |
| 16a | n-$C_3H_7$ | 1-Naphthyl |
| 17a | n-$C_3H_7$ | 2-Naphthyl |
| 18a | n-$C_3H_7$ | 4-Biphenyl |
| 19a | n-Bu | Phenyl |
| 20a | n-Bu | 4-Cl-Ph |
| 21a | n-Bu | 4-F-Ph |
| 22a | n-Bu | 4-Me-Ph |
| 23a | n-Bu | 2-Cl-Ph |
| 24a | n-Bu | 2-F-Ph |
| 25a | n-Bu | 2,4-$Cl_2$-Ph |
| 26a | n-Bu | 3-Pyridin |
| 27a | t-Bu | Ph |
| 28a | t-Bu | 4-Cl-Ph |
| 29a | t-Bu | 4-Cl-Ph |
| 30a | t-Bu | 4-Cl-Ph |
| 31a | t-Bu | 4-Cl-Ph |
| 32a | t-Bu | 4-Cl-Ph |
| 33a | t-Bu | 4-Cl-Ph |

Tabelle 2 (Fortsetzung)

| Nr. | $R^1$ | $R^2$ | |
|---|---|---|---|
| 34a | t-Bu | 4-F-Ph | |
| 35a | t-Bu | 4-F-Ph | |
| 36a | t-Bu | 4-Me-Ph | |
| 37a | t-Bu | 2-Cl-Ph | |
| 38a | t-Bu | 2-F-Ph | |
| 39a | t-Bu | 2,4-$Cl_2$-Ph | |
| 40a | t-Bu | 3-Pyridin | |
| 41a | t-Bu | 3-Pyridin | |
| 42a | t-Bu | Cyclohexyl | |
| 43a | t-Bu | Cyclohexyl | |
| 44a | t-Bu | Cyclohexyl | |
| 45a | t-Bu | Cyclohexyl | |
| 46a | t-Bu | Cyclohexyl | |
| 47a | t-Bu | Cyclohexyl | |
| 48a | t-Bu | Cyclohexyl | |
| 49a | n-Pentyl | Ph | |
| 50a | n-Pentyl | 4-Cl-Ph | |
| 51a | n-Pentyl | 4-F-Ph | |
| 52a | n-Pentyl | 4-Me-Ph | |
| 53a | n-Pentyl | 2-Cl-Ph | |
| 54a | n-Pentyl | 2-F-Ph | |
| 55a | n-Pentyl | 2,4-$Cl_2$-PH | |
| 56a | n-Pentyl | 3-Pyridin | |
| 57a | n-Pentyl | 3-Pyridin | |
| 58a | n-Pentyl | 3-Pyridin | |
| 59a | n-Pentyl | 3-Pyridin | |
| 60a | n-Pentyl | 3-Pyridin | |
| 61a | n-Pentyl | 3-Pyridin | |
| 62a | $CH_2$+ | Ph | |
| 63a | $CH_2$+ | 4-Cl-Ph | Kp 117-120°C/0,4 mbar |
| 64a | $CH_2$+ | 4-F-Ph | |
| 65a | $CH_2$+ | 4-Me-Ph | |
| 66a | $CH_2$+ | 2-Cl-Ph | |
| 67a | $CH_2$+ | 2-F-Ph | |
| 68a | $CH_2$+ | 2,4-$Cl_2$-Ph | |
| 69a | $CH_2$+ | 3-Pyridin | |
| 70a | n-Hexyl | Ph | |
| 71a | n-Hexyl | 4-Cl-Ph | |
| 72a | n-Hexyl | 4-F-Ph | |

Tabelle 2 (Fortsetzung)

| Nr. | $R^1$ | $R^2$ | |
|---|---|---|---|
| 73a | n-Hexyl | 4-Me-Ph | |
| 74a | n-Hexyl | 2-Cl-Ph | |
| 75a | n-Hexyl | 2-F-Ph | |
| 76a | n-Hexyl | 2,4-$Cl_2$-Ph | |
| 77a | n-Hexyl | 3-Pyridin | |
| 78a | $CH_2-CH_2$+ | Ph | |
| 79a | $CH_2-CH_2$+ | 4-Cl-Ph | |
| 80a | $CH_2-CH_2$+ | 4-F-Ph | |
| 81a | $CH_2-CH_2$+ | 4-Me-Ph | |
| 82a | $CH_2-CH_2$+ | 2-Cl-Ph | |
| 83a | $CH_2-CH_2$+ | 2-F-Ph | |
| 84a | $CH_2-CH_2$+ | 2,4-$Cl_2$-Ph | |
| 85a | $CH_2-CH_2$+ | 3-Pyridin | |
| 86a | Ph | Ph | |
| 87a | Ph | 4-Cl-Ph | |
| 88a | Ph | 4-F-Ph | |
| 89a | Ph | 4-Me-Ph | |
| 90a | Ph | 2-Cl-Ph | |
| 91a | Ph | 2-F-Ph | |
| 92a | Ph | 2,4-$Cl_2$-Ph | |
| 93a | Ph | 3-Pyridin | Fp 94°C |
| 94a | Ph | n-Bu | |
| 95a | Ph | t-Bu | |
| 96a | Ph | n-Pentyl | |
| 97a | Ph | $CH_2$+ | |
| 98a | Ph | $CH_2$+ | |
| 99a | Ph | $CH_2$+ | |
| 100a | Ph | $CH_2$+ | |
| 101a | Ph | $CH_2$+ | |
| 102a | Ph | $CH_2$+ | |
| 103a | Ph | $CH_2$+ | |
| 104a | 4-Cl-Ph | $CH_2$+ | |
| 105a | Ph | n-Hexyl | |
| 106a | Ph | $CH_2-CH_2$+ | |
| 107a | Ph | $CH_2-CH_2$+ | |
| 108a | Ph | $CH_2-CH_2$+ | |
| 109a | Ph | $CH_2-CH_2$+ | |
| 110a | Ph | $CH_2-CH_2$+ | |
| 111a | Ph | $CH_2-CH_2$+ | |

Tabelle 2 (Fortsetzung)

| Nr. | $R^1$ | $R^2$ | |
|---|---|---|---|
| 112a | Ph | $CH_2$-$CH_2$ | |
| 113a | Ph | $CH_2$-$CH_2$+ | |
| 114a | Ph | $CH_2$-$CH_2$-Ph | |
| 115a | Ph | $CH_2$-$CH_2$-Ph | |
| 116a | Ph | $CH_2$-$CH_2$-Ph | |
| 117a | Ph | $CH_2$-$CH_2$-Ph | |
| 118a | Ph | $CH_2$-$CH_2$-Ph | |
| 119a | Ph | $CH_2$-$CH_2$-Ph | |
| 120a | Ph | $CH_2$-$CH_2$-Ph | |
| 121a | Ph | $CH_2$-$CH_2$-Ph | |
| 122a | 4-Cl-Ph | Ph | |
| 123a | 4-Cl-Ph | 4-Cl-Ph | |
| 124a | 4-Cl-Ph | 4-F-Ph | |
| 125a | 4-Cl-Ph | 4-F-Ph | |
| 126a | 4-Cl-Ph | 4-F-Ph | |
| 127a | 4-Cl-Ph | 4-F-Ph | |
| 128a | 4-Cl-Ph | 4-F-Ph | |
| 129a | 4-Cl-Ph | 4-F-Ph | |
| 130a | 4-Cl-Ph | 4-F-Ph | |
| 131a | 4-Cl-Ph | 4-F-Ph | |
| 132a | 4-Cl-Ph | 4-Me-Ph | |
| 133a | 4-Cl-Ph | 2-Cl-Ph | |
| 134a | 4-Cl-Ph | 2-F-Ph | |
| 135a | 4-Cl-Ph | 2,4-$Cl_2$-Ph | |
| 136a | 4-Cl-Ph | $CH_2$+ | Kp 119-122°C/0,01 mbar |
| 137a | 4-Cl-Ph | $CH_2$+ | |
| 138a | 4-F-Ph | 2-Cl-Ph | Kp 164-184°C/0,3 mbar |
| 139a | 4-Cl-Ph | 2,2-$(CH_3)_2$-n-propyl | Kp 120°C/0,01 mbar |
| 140a | 4-F-Ph | 2,2-$(CH_3)_2$-n-propyl | Kp 85°C/0,1 mbar |
| 141a | 4-Cl-Ph | -$CH_2$-$CH_2$-CH-$(CH_3)_2$-$CH_3$ | Kp 120°C/0,1 mbar |
| 143a | 1,1-$(CH_3)_2$-n-ethyl | 4-Cl-Ph | Kp 120°C/0,4 mbar |
| 144a | 4-F-Ph | 3-Pyridin | Kp 155°C/0,1 mbar |
| 145a | 4-Cl-Ph | 4-F-Ph | Fp 84-85,5°C |
| 146a | 4-Cl-Ph | 2,4-Dichlor-Ph | Fp 130,5-131,5°C |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,

Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je kg Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 109 (Tab. 1) mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 136 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 139 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 159 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen

des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 168 werden mit 3 Gew.-Teilen des Natriumsalzes der Diisobutylnaphthalin-$\alpha$-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 174 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 109 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 136 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 139 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiel

Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inoculiert. Anschließend wurden die Versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

Das Ergebnis zeigt, daß die Wirkstoffe 109, 136, 139, 159, 168 und 174 bei der Anwendung als 0,05 %ige (Gew.%) Spritzbrühe eine gute fungizide Wirkung zeigen (90 %).

## Patentansprüche

**1.** 1-Aza-butadiene der Formel I

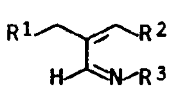

I

in denen

$R^1$ und $R^2$ Phenyl, Biphenyl, Naphthyl oder Pyridyl bedeuten, wobei diese Reste einfach bis dreifach durch Halogen, Nitro, Phenoxy, Amino, Haloalkyl, Alkyl oder Alkoxy mit jeweils 1 bis 4 C-Atomen substituiert sein können, oder

einen $C_1$-$C_8$-Alkylrest bedeuten, der durch einen gegebenenfalls substituierten Phenylrest substituiert sein kann,

$R^3$ einen gegebenenfalls substituierten Phenylrest oder den Rest $OR^4$ bedeutet,

$R^4$ Wasserstoff, einen C1-$C_6$-Alkylrest oder eine aromatische oder aliphatische Acylgrupe bedeutet oder $R^3$ den Rest $NHR^5$ bedeutet,

$R^5$ Wasserstoff oder einen gegebenenfalls substituierten Phenylrest oder die Carbamid-Gruppe $CONH_2$ bedeutet. Und zusätzlich die Verbindungen, in denen $R^1$ tertiär-Butyl, $R^2$ Cyclohexyl und $R^3$ OH, OMe, OEt, O-CO-Me, O-CO-Phenyl, NH-Phenyl oder 4-Br-Phenyl bedeuten.

2. Verbindungen der Formel I gemäß Anspruch 1, in denen $R^2$ eine 2,2-Dimethylpropylgruppe, $R^3$ die OH-Gruppe und $R^1$ einen Phenylrest bedeuten, der durch einen oder zwei gleiche oder verschiedene Reste Fluor, Chlor, Brom oder Methyl substituiert sein kann.

3. Verfahren zur Herstellung der Verbindungen der Formel I gemäß Anspruch 1, dadurch gekennzeichnet, daß man Verbindungen der Formel II,

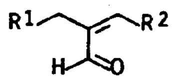

II .

in denen $R^1$ bis $R^3$ die im Anspruch 1 genannten Bedeutungen haben, mit Verbindungen der Formel $H_2NR^3$ umsetzt.

4. Fungizides Mittel enthaltend einen Trägerstoff und eine fungizid wirksame Menge eines 1-Aza-butadiens der Formel I

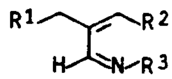

I

in denen
$R^1$ und $R^2$ Phenyl, Biphenyl, Naphthyl oder Pyridyl bedeuten, wobei diese Reste einfach bis dreifach durch Halogen, Nitro, Phenoxy, Amino, Haloalkyl, Alkyl oder Alkoxy mit jeweils 1 bis 4 C-Atomen substituiert sein können, oder
einen $C_1$-$C_8$-Alkylrest bedeuten, der durch einen gegebenenfalls substituierten Phenylrest substituiert sein kann,
$R^3$ einen gegebenenfalls substituierten Phenylrest oder den Rest $OR^4$ bedeutet,
$R^4$ Wasserstoff, einen $C_1$-$C_6$-Alkylrest oder eine aromatische oder aliphatische Acylgrupe bedeutet oder $R^3$ den Rest $NHR^5$ bedeutet,
$R^5$ Wasserstoff oder einen gegebenenfalls substituierten Phenylrest oder die Carbamid-Gruppe $CONH_2$ bedeutet, und zusätzlich die Verbindungen, in denen $R^1$ tertiär-Butyl, $R^2$ Cyclohexyl und $R^3$ OH, OMe, OEt, O-CO-Me, O-CO-Phenyl, NH-Phenyl oder 4-Br-Phenyl bedeuten.

5. Verfahren zur Bekämpfung von Pilzen, dadurch gekennzeichnet, daß man die Pilze oder die von Pilzbefall bedrohten Pflanzen, Materialien, Saatgüter oder den Erdboden behandelt mit einer fungizid wirksamen Menge eines 1-Aza-butadiens der Formel I

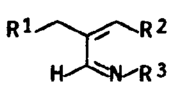

I

in denen
$R^1$ und $R^2$ Phenyl, Biphenyl, Naphthyl oder Pyridyl bedeuten, wobei diese Reste einfach bis dreifach durch Halogen, Nitro, Phenoxy, Amino, Haloalkyl, Alkyl oder Alkoxy mit jeweils 1 bis 4 C-Atomen substituiert sein können, oder
einen $C_1$-$C_8$-Alkylrest bedeuten, der durch einen gegebenenfalls substituierten Phenylrest substituiert sein kann,
$R^3$ einen gegebenenfalls substituierten Phenylrest oder den Rest $OR^4$ bedeutet,
$R^4$ Wasserstoff, einen $C_1$-$C_6$-Alkylrest oder eine aromatische oder aliphatische Acylgrupe bedeutet oder $R^3$ den Rest $NHR^5$ bedeutet,
$R^5$ Wasserstoff oder einen gegebenenfalls substituierten Phenylrest oder die Carbamid-Gruppe $CONH_2$ bedeutet, und zusätzlich die Verbindungen, in denen $R^1$ tertiär-Butyl, $R^2$ Cyclohexyl und $R^3$ OH, OMe, OEt, O-CO-Me, O-CO-Phenyl, NH-Phenyl oder 4-Br-Phenyl bedeuten.

6. Verbindung der Formel II

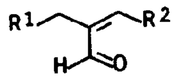

II

in der $R^1$ und $R^2$ Phenyl, Biphenyl, Naphthyl oder Pyridyl bedeuten, wobei diese Reste einfach bis dreifach durch Halogen, Nitro, Phenoxy, Amino, Haloalkyl, Alkyl oder Alkoxy mit jeweils 1 bis 4 C-Atomen substituiert sein können, oder einen $C_1$-$C_8$-Alkylrest bedeuten, der durch einen gegebenenfalls substituierten Phenylrest substituiert sein kann, mit der Maßgabe, daß nur einer der beiden Reste $R^1$ und $R^2$ einen $C_1$-$C_8$-Alkylrest bedeutet und zusätzlich die Verbindung, in der $R^1$ tertiär-Butyl und $R^2$ Cyclohexyl bedeuten.

**7.** Verbindung der Formel I gemäß Anspruch 1, dadurch gekennzeichnet, daß $R^1$ Phenyl, $R^2$ 3,3-Dimethylbutyl und $R^3$ Acetyloxy bedeutet.

**8.** Verbindung der Formel I gemäp Anspruch 1, dadurch gekennzeichnet, daß $R^1$ 4-Chlorphenyl, $R^2$ 2,2-Dimethylpropyl und $R^3$ OH bedeutet.

**9.** Verbindung der Formel I gemäß Anspruch 1, dadurch gekennzeichnet, daß $R^1$ 4-Chlorphenyl, $R^2$ 2,2-Dimethylpropyl und $R^3$ Acetyloxy bedeutet.

## Claims

**1.** A 1-azabutadiene of the formula I

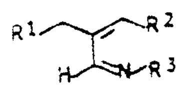

where $R^1$ and $R^2$ are each phenyl, biphenylyl naphthyl or pyridyl, and these radicals may be monosubstituted to trisubstituted by halogen, nitro, phenoxy, amino, haloalkyl, alkyl or alkoxy, each of 1 to 4 carbon atoms, or are each $C_1$-$C_8$-alkyl which may be substituted by substituted or unsubstituted phenyl, where $R^3$ is substituted or unsubstituted phenyl or a radical $OR^4$, where $R^4$ is hydrogen, $C_1$-$C_6$-alkyl or an aromatic or aliphatic acyl groups or $R^3$ is a radical $NHR^5$, where $R^5$ is hydrogen, substituted or unsubstituted phenyl or the carbamide group $CONH_2$ and, in addition, where $R^1$ is tert-butyl, $R^2$ is cyclohexyl and $R^3$ is OH, OMe, OEt, O-CO-Me, O-CO-phenyl, NH-phenyl or 4-Br-phenyl.

**2.** A compound of the formula I as claimed in claim 1, where $R^2$ is 2,2-dimethylpropyl, $R^3$ is OH and $R^1$ is phenyl which may be substituted by one or two identical or different radicals selected from the group consisting of fluorine, chlorine, bromine and methyl.

**3.** A process for preparing a compound of the formula I as claimed in claim 1, which comprises reacting a compound of the formula II

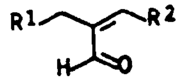

(II)

where $R^1$ to $R^3$ are as defined in claim 1, with a compound of the formula $H_2NR^3$.

**4.** A fungicidal agent containing a carrier and a fungicidally effective amount of a 1-azabutadiene of the formula I

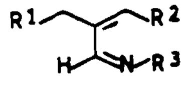

(I)

where $R^1$ and $R^2$ are each phenyl, biphenylyl, naphthyl or pyridyl, and these radicals may be monosubstituted to trisubstituted by halogen, nitro, phenoxy, amino, haloalkyl, alkyl or alkoxy, each of 1 to 4 carbon atoms, or are each $C_1$-$C_8$-alkyl which may be substituted by substituted or unsubstituted phenyl, where $R^3$ is substituted or unsubstituted phenyl or a radical $OR^4$, where $R^4$ is hydrogen, $C_1$-$C_6$-alkyl or an aromatic or aliphatic acyl group, or $R^3$ is a radical $NHR^5$, where $R^5$ is hydrogen, substituted or unsubstituted phenyl or the carbamide group $CONH_2$, and, in addition, where $R^1$ is tert-butyl, $R^2$ is cyclohexyl and $R^3$ is OH, OMe, OEt, O-CO-Me, O-CO-phenyl, NH-phenyl or 4-Br-phenyl.

5. A method for controlling fungi, wherein the fungi or the plants, materials, seed or the soil threatened by fungal attack are treated with a fungicidally effective amount of a 1-azabutadiene of the formula I

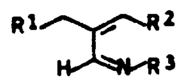

I

where $R^1$ and $R^2$ are each phenyl, biphenyl, naphthyl or pyridyl, and these radicals may be monosubstituted to trisubstituted by halogen, nitro, phenoxy, amino, haloalkyl, alkyl or alkoxy, each of 1 to 4 carbon atoms, or are each $C_1$-$C_8$-alkyl which may be substituted by substituted or unsubstituted phenyl, where $R^3$ is substituted or unsubstituted phenyl or a radical $OR^4$, where $R^4$ is hydrogen, $C_1$-$C_6$-alkyl or an aromatic or aliphatic acyl group, or $R^3$ is a radical $NHR^5$ where $R^5$ is hydrogen, substituted or unsubstituted phenyl or the carbamide group $CONH_2$, and, in addition, $R^1$ is tert-butyl, $R^2$ is cyclohexyl and $R^3$ is OH, OMe, OEt, O-CO-Me, O-CO-phenyl, NH-phenyl or 4-Br-phenyl.

6. A compound of the formula II

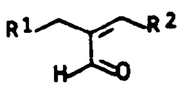

II

where $R^1$ and $R^2$ are each phenyl, biphenylyl, naphthyl or pyridyl, and these radicals may be monosubstituted to trisubstituted by halogen, nitro, phenoxy, amino, haloalkyl, alkyl or alkoxy, each of 1 to 4 carbon atoms, or are each $C_1$-$C_8$-alkyl which may be substituted by substituted or unsubstituted phenyl, with the proviso that only one of the two radicals $R^1$ and $R^2$ is $C_1$-$C_8$-alkyl, and, in addition, where $R^1$ is tert-butyl and $R^2$ is cyclohexyl.

7. A compound of the formula I as claimed in claim 1, wherein $R^1$ is phenyl, $R^2$ is 3,3-dimethylbutyl and $R^3$ is acetyloxy.

8. A compound of the formula I as claimed in claim 1, wherein $R^1$ is 4-chlorophenyl, $R^2$ is 2,2-dimethylpropyl and $R^3$ is OH.

9. A compound of the formula I as claimed in claim 1, wherein $R^1$ is 4-chlorophenyl, $R^2$ is 2,2-dimethylpropyl and $R^3$ is acetyloxy.

## Revendications

1. 1-Aza-butadiènes de formule I

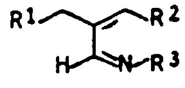

I,

dans laquelle
$R^1$ et $R^2$ représentent phényle, biphényle, naphtyle ou pyridyle, ces restes pouvant être substitués une à trois fois par halogène, nitro, phénoxy, amino, haloalkyle, alkyle ou alcoxy à 1 à 4 atomes C dans chaque

cas, ou
un reste alkyle en $C_1$-$C_8$, qui peut être substitué par un reste phényle éventuellement substitué,
$R^3$ représente un reste phényle éventuellement substitué ou le reste $OR^4$,
$R^4$ représente hydrogène, un reste alkyle en $C_1$-$C_6$ ou un groupe acyle aromatique ou aliphatique ou $R^3$ le reste $NHR^5$,
$R^5$ représente hydrogène ou un reste phényle éventuellement substitué ou le groupe carbamide $CONH_2$. Et additionnellement les composés dans lesquels $R^1$ représente ter-butyle, $R^2$ cyclohexyle et $R^3$ OH, OMe, OEt, O-CO-Me, O-CO-phényle, NH-phényle ou 4-Br-phényle.

2. Composés de formule I, selon la revendication 1, dans lesquels $R^2$ représente un groupe 2,2-diméthylpropyle, $R^3$ le groupe OH et $R^1$ un reste phényle qui peut être substitué par un ou deux restes fluor, chlore, brome ou méthyle identiques ou différents.

3. Procédé de préparation des composés de formule I, selon la revendication 1, caractérisé par le fait que l'on fait réagir des composés de formule II

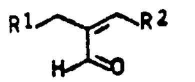

II,

dans laquelle $R^1$ à $R^3$ ont les significations données dans la revendication 1, avec des composés de formule $H_2NR^3$.

4. Fongicide, contenant un véhicule et une quantité efficace au point de vue fongicide d'un 1-aza-butadiène de formule I

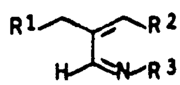

I,

dans laquelle
$R^1$ et $R^2$ représentent phényle, biphényle, naphtyle ou pyridyle, ces restes pouvant être substitués une à trois fois par halogène, nitro, phénoxy, amino, haloalkyle, alkyle ou alcoxy à 1 à 4 atomes C dans chaque cas, ou
un reste alkyle en $C_1$-$C_8$, qui peut être substitué par un reste phényle éventuellement substitué,
$R^3$ représente un reste phényle éventuellement substitué ou le reste $OR^4$,
$R^4$ représente hydrogène, un reste alkyle en $C_1$-$C_6$ ou un groupe acyle aromatique ou aliphatique ou $R^3$ le reste $NHR^5$,
$R^5$ représente hydrogène ou un reste phényle éventuellement substitué ou le groupe carbamide $CONH_2$ et additionnellement les composés dans lesquels $R^1$ représente ter-butyle, $R^2$ cyclohexyle et $R^3$ OH, OMe, OEt, O-CO-Me, O-CO-phényle, NH-phényle ou 4-Br)phényle.

5. Procédé de lutte contre les champignons, caractérisé par le fait que l'on traite les champignons ou les plantes, matériaux, semences ou le sol menacés par l'attaque par les champignons, avec une quantité efficace au point de vue fongicide d'un 1-aza-butadiène de formule I

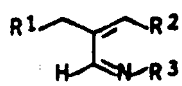

I,

dans laquelle
$R^1$, $R^2$, représentent phényle, biphényle, naphtyle ou pyridyle, ces restes pouvant être substitués une à trois fois par halogène, nitro, phénoxy, amino, haloalkyle, alkyle ou alcoxy à 1 à 4 atomes C dans chaque cas, ou
un reste alkyle en $C_1$-$C_8$, qui peut être substitué par un reste phényle éventuellement substitué,
$R^3$ représente un reste phényle éventuellement substitué ou le reste $OR^4$,

$R^4$ représente hydrogène, un reste alkyle en $C_1$-$C_6$ ou un groupe acyle aromatique ou aliphatique ou $R^3$ le reste $NHR^5$,
$R^5$ représente hydrogène ou un reste phényle éventuellement substitué ou le groupe carbamide $CONH_2$.
Et additionnellement les composés dans lesquels $R^1$ représente ter-butyle, $R^2$ cyclohexyle et $R^3$ OH, OMe, OEt, O-CO-Me, O-CO-phényle, NH-phényle ou 4-Br-phényle.

6. Composé de formule II

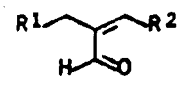

II,

dans laquelle $R^1$ et $R^2$ représentent phényle, biphényle, naphtyle ou pyridyle, ces restes pouvant être substitués une à trois fois par halogène, nitro, phénoxy, amino, haloalkyle, alkyle ou alcoxy à 1 à 4 atomes C dans chaque cas, ou un reste alkyle en $C_1$-$C_8$ qui peut être substitué par un reste phényle éventuellement substitué, étant entendu que seulement un des deux restes $R^1$ et $R^2$ représente un reste alkyle en $C_1$-$C_8$, et, additionnellement, le composé dans lequel $R^1$ représente ter-butyle et $R^2$ cyclohexyle.

7. Composé de formule I, selon la revendication 1, caractérisé par le fait que $R^1$ représente phényle, $R^2$, 3,3-diméthylbutyle et $R^3$ acétyloxy.

8. Composé de formule I, selon la revendication 1, caractérisé par le fait que $R^1$ représente 4-chlorophényle, $R^2$ 2,2-diméthylpropyle et $R^3$ OH.

9. Composé de formule I, selon la revendication 1, caractérisé par le fait que $R^1$ représente 4-chlorophényle, $R^2$ 2,2-diméthylpropyle et $R^3$ acétyloxy.